# FASCICULE DE BREVET EUROPEEN

(11) **EP 1 068 852 B1**
(45) Date de publication et mention de la délivrance du brevet: **08.09.2004**
(21) Numéro de dépôt: 00401575.6
(22) Date de dépôt: 05.06.2000
(51) Int. Cl.: A61K 7/00, B01F 17/00

(54) **Composition solide et ses utilisations notamment cosmétiques**
Feste Zusammensetzung und ihre Verwendungen insbesondere in kosmetische Verwendungen
Solid composition and its use especially in cosmetics

(30) Priorité: 12.07.1999 FR 9909013
(43) Date de publication de la demande: 17.01.2001
(73) Titulaire: L'OREAL, 75008 Paris (FR)
(72) Inventeur: Afriat, Isabelle, 75003 Paris (FR); Boulier, Virginie, 95520 Osny (FR)
(74) Mandataire: Rasson, Catherine

(56) Documents cités:
- EP-A- 0 612 517
- EP-A- 0 670 157
- EP-A- 0 965 331
- EP-A- 0 970 682
- WO-A-93/14742
- WO-A-95/15812
- WO-A-99/47111

## Description

L'invention se rapporte à une composition solide se présentant sous forme d'une émulsion eau-dans-huile (E/H) contenant une grande quantité de phase aqueuse, au moins une cire et un émulsionnant siliconé, et les utilisations de ladite composition notamment dans les domaines cosmétique et/ou dermatologique.

Dans le domaine cosmétique, la fraîcheur des produits appliqués sur la peau est une préoccupation importante, dans les pays chauds ou bien en été où le besoin de textures rafraîchissantes est important.

De manière classique, on obtient des compositions donnant une impression de fraîcheur en utilisant des gels aqueux ou des émulsions huile-dans-eau dont la phase externe est aqueuse. En effet, avec les gels aqueux ou avec les émulsions comportant une phase aqueuse externe, l'effet immédiat ressenti lors de l'application sur la peau est apporté par l'eau qui s'évapore immédiatement donnant ainsi une impression de fraîcheur.

Par ailleurs, les actifs présents dans une émulsion présentent une meilleure efficacité lorsqu'ils sont dans la phase dispersée de l'émulsion. Ainsi, les émulsions à phase continue aqueuse (huile-dans-eau ou H/E) permettent une meilleure efficacité des actifs lipophiles, tandis que les émulsions à phase continue huileuse (eau-dans-huile ou E/H) permettent une meilleure efficacité des actifs hydrophiles. Il est donc intéressant de disposer aussi d'émulsions E/H. Toutefois, les émulsions E/H présentent l'inconvénient d'être inconfortables du fait de la sensation grasse et lourde apportée par la phase grasse externe qui subsiste sur la peau. Ainsi, ces émulsions n'apportent pas de fraîcheur et sont généralement trop riches en huiles pour être utilisées pendant l'été ou dans les pays chauds.

Pour surmonter ces inconvénients, il a été envisagé de préparer des émulsions E/H à forte teneur en eau. Toutefois, la teneur en eau ne peut pas être trop importante pour des raisons de stabilité, ou alors une forte teneur en eau doit être compensée par l'ajout de plusieurs tensioactifs ou d'agents gélifiants qui peuvent nuire au confort de la composition finale et même entraîner des problèmes d'irritations cutanés notamment chez les sujets à peaux sensibles.

Il subsiste donc le besoin d'une émulsion eau-dans-huile stable, comportant une quantité importante d'eau et utilisable dans les domaines cosmétique et/ou dermatologique, qui ne présente pas les inconvénients de l'art antérieur.

La demanderesse a maintenant trouvé une composition solide du type émulsion eau dans huile permettant d'atteindre ces objectifs, et notamment donnant une grande impression de fraîcheur bien qu'ayant une phase continue huileuse.

La présente invention a pour objet une composition solide comprenant, dans un milieu physiologiquement acceptable, une phase aqueuse dispersée dans une phase huileuse, caractérisée en ce que la phase aqueuse représente au moins 70 % en poids par rapport au poids total de la composition, en ce que l'eau représente au moins 70% en poids de la composition, et en ce que la composition comprend au moins un émulsionnant siliconé et au moins 3 % en poids d'au moins une cire, par rapport au poids total de la composition.

On entend par milieu physiologiquement acceptable dans la composition de l'invention, un milieu non toxique et susceptible d'être appliqué sur la peau (y compris l'intérieur des paupières) ou les lèvres d'êtres humains.

Par ailleurs, on entend par « composition solide » une composition présentant une résistance à la compression supérieure ou égale à 50 grammes, à température ambiante, après pénétration par une sonde cylindrique de révolution ayant un diamètre de 0,8 cm dans la matrice de la composition dans une épaisseur de 5 mm, à une vitesse de 1 mm/s, maintien de ladite sonde dans la matrice de la composition pendant 15 secondes et retrait de ladite sonde de la matrice de la composition à une vitesse de 1mm/s ; la résistance à la compression étant mesurée avec un analyseur de texture du type TAXT2 commercialisé par la société RHEO.

En dépit de la quantité importante d'eau, la composition de l'invention est stable dans le temps.

La composition selon l'invention comporte au moins 70 % en poids de phase aqueuse par rapport au poids total de la composition et de préférence au moins 75 % du poids total de la composition. La phase aqueuse peut constituer jusqu'à 92 % du poids total de la composition.

L'eau cbnstitue au moins 70 % du poids total de la composition.

Par ailleurs, la phase aqueuse de l'émulsion peut contenir un ou plusieurs alcools inférieurs tels que l'éthanol en une quantité allant de préférence jusqu'à 15 % et mieux jusqu'à 10 % du poids total de la composition, et/ou un ou plusieurs polyols tels que la glycérine et le propylène glycol, en une quantité allant par exemple jusqu'à 20 % et mieux jusqu'à 10 % du poids total de la composition.

La composition de l'invention contient, comme agent émulsionnant, un émulsionnant siliconé. Celui-ci a généralement un HLB (Hydrophilic Lipophilic Balance) inférieur à 8 et peut être choisi notamment parmi les diméthicone-copolyols, les alkyl- ou alcoxy-diméthicone copolyols et les organopolysiloxanes solides élastomères réticulés comportant au moins un groupement oxyalkyléné.

Comme diméthicone copolyol utilisable dans l'émulsion selon l'invention, on peut citer par exemple les mélanges suivants commercialisés par la société Dow Corning :
- mélange de diméthicone copolyol, de tétracyclométhicone (D4) et d'eau (rapport pondéral 10/88/2), commercialisé sous la dénomination DC 3225C ;
- mélange de diméthicone copolyol, de pentacyclométhicone (D5) et d'eau (rapport pondéral 10/88/2), commercialisé sous la dénomination DC 5225C ;
- mélange de diméthicone copolyol et de polydiméthylsiloxane 5 cSt (rapport pondéral 10/90), commercialisé sous la dénomination DC 3225C in 200 Fluid 5 cSt;
- mélange de diméthicone copolyol et de polydiméthylsiloxane 10 cSt (rapport pondéral 10/90), commercialisé sous la dénomination DC 3225C in 200 Fluid 10 cSt;
- mélange de diméthicone copolyol et de pentacyclométhicone (D5) (rapport pondéral 43/57), commercialisé sous la dénomination DC 5185C,
ainsi que le produit vendu sous la dénomination "SF-1228" par la Société GENERAL ELECTRIC.

Comme alkyldiméthiconecopolyol, on peut utiliser par exemple le lauryl-diméthicone-copolyol comme celui vendu sous la dénomination Q2-5200 par la Société Dow Corning, le cétyldiméthiconecopolyol comme celui vendu sous la dénomination ABIL EM90 par la Société Goldschmidt ou comme le mélange polyglycéryl-4 isostéarate/ cétyldiméthicone copolyol/ hexyl laurate vendu sous le nom d'Abil WE 09 par la société Goldschmidt, l'oleyldiméthicone copoylol comme celui vendu sous la dénomination KF-6026 par la Société Shin-Etsu, le stéaryldiméthicone copolyol comme celui vendu sous la dénomination X-22-904 par la Société Shin-Etsu.

Dans l'expression « organopolysiloxanes solides élastomères réticulés comportant au moins un groupement oxyalkyléné », on entend par « élastomère » un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes solides élastomères réticulés utilisables dans la composition de l'invention contiennent un ou plusieurs groupements oxyalkylénés et en particulier oxyéthylénés (OE), par exemple de 1 à 40 motifs oxyalkylénés et mieux 1 à 20 motifs oxyalkylénés, pouvant former des chaînes polyoxyalkylènes et notamment polyoxyéthylènes. Ces groupements peuvent être pendants, en bout de chaîne ou destinés à lier deux parties de la structure siliconée. Les atomes de silicium portant ces groupements sont au nombre d'environ 1 à 10.

Bien que l'invention concerne plus spécialement les organopolysiloxanes solides élastomères réticulés à groupement(s) oxyéthyléné(s), elle peut aussi concerner les organopolysiloxanes à groupement(s) oxypropyléné(s). Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Les organopolysiloxanes solides élastomères réticulés utilisables dans la composition de l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse en un gel homogène, en présence de quantités de phase huileuse plus élevées. La gélification de la phase huileuse par ces élastomères peut être totale ou partielle.

Ces organopolysiloxanes peuvent se présenter sous forme de poudre, les particules constituant cette poudre ayant une taille allant de 0,1 à 500 pm, et mieux de 3 à 200 µm, et pouvant être sphériques, plates ou amorphes avec, de préférence, une forme sphérique. Ils peuvent aussi se présenter sous forme de gel contenant l'organopolysiloxane élastomère dispersé dans une phase huileuse. Cette phase huileuse, appelée encore phase grasse liquide, peut comprendre tout corps non aqueux ou mélange de corps non aqueux, liquide à température ambiante (25°C).

Ces organopolysiloxanes élastomères peuvent être choisis parmi les polymères réticulés obtenus par réaction d'addition et de réticulation en milieu non aqueux, en présence d'un catalyseur du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins un groupement oxyalkyléné notamment oxyéthyléné.

En particulier, l'organopolysiloxane (i) est choisi parmi les polydiméthylsiloxanes (PDMS) et est plus spécifiquement un α-ω-diméthylvinyl polydiméthylsiloxane. L'organopolysiloxane (ii) est choisi notamment parmi les polydiméthylsiloxanes comportant un ou plusieurs atome(s) d'hydrogène lié chacun à un atome de silicium, et un ou plusieurs groupements oxyéthylénés et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Comme indiqué ci-dessus, les organopolysiloxanes élastomères utilisables dans la composition selon l'invention se présentent avantageusement dans une phase huileuse avec laquelle ils constituent un gel anhydre. Ce gel peut être notamment obtenu comme suit :
- (a) mélange du premier organopolysiloxane (i) et du second organopolysiloxane (ii);
- (b) ajout d'une phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les PDMS à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes de l'invention sont en particulier obtenus selon le mode opératoire des exemples 3, 4 et 8 du document US-A-5,412,004 et des exemples du document US-A-5,811,487.

Les organopolysiloxanes élastomères utilisables dans la composition de l'invention sont par exemple celui commercialisé sous la référence KSG 21 par la société Shin Etsu ou le produit de l'exemple 3 (exemple de synthèse) du brevet US-A-5,412,004.

Le KSG 21 se présente sous forme d'un gel et comprend 28 % d'organopolysiloxane et 72 % d'huile de silicone (PDMS) ayant une viscosité de 6 cSt.

Le produit de l'exemple 3 (exemple de synthèse) du document US-A-5,412,004 se présente sous la forme d'un gel pâteux contenant environ 32 à 33 % en poids d'organopolysiloxane réticulé à groupement(s) oxyéthyléné(s) et environ 67 à 68 % de PDMS 6 cSt. L'organopolysiloxane contient environ 18 % d'oxyde d'éthylène en poids par rapport au poids total du polymère. Ce gel élastomère a un comportement rhéofluidifiant plastique de 2.10⁶ poises à 4.10⁶ poises et une viscosité dynamique de 45 poises pour une vitesse de cisaillement de 200 s⁻¹, mesurée avec un rhéomètre à contrainte imposée, RS 75 (Haake) à 25°C en géométrie cône/plan ; caractéristiques du cône : 20 mm de diamètre, 1° d'angle et 40 µm de gap. Cet organopolysiloxane a, en outre, un comportement viscoélastique avec un caractère élastique dominant aux faibles valeurs de la contrainte de cisaillement, défini comme suit : 800 Pa < G *ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité. Il présente un point éclair d'environ 170°C à la pression atmosphérique.

L'émulsionnant siliconé est présent dans la composition de l'invention, de préférence en une quantité de matière active allant de 0,1 à 10 %, mieux de 0,3 à 5 % et encore mieux de 0,4 à 2,5 % en poids par rapport au poids total de la composition.

Le rapport pondéral phase huileuse/émulsionnant siliconé est de préférence égal ou supérieur à 5 et mieux égal ou supérieur à 8.

La composition comprend au moins une cire. Les cires utilisables dans la composition de l'invention peuvent être choisies parmi les cires d'origine animale, telles que la cire d'abeilles, le spermaceti, la cire de lanoline et les dérivés de lanoline; les cires d'origine végétale, telles que la cire de Carnauba, de Candellila, d'Ouricury, du Japon, le beurre de cacao ou les cires de fibres de liège ou de canne à sucre ; les cires minérales, par exemple de paraffine, de vaseline, de lignite, ou les cires microcristallines ou les ozokérites ; les cires synthétiques parmi lesquelles les cires de polyéthylène, de polytétrafluoroéthylène et les cires obtenues par synthèse de Fischer-Tropsch ; les cires de silicone ; les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C20-C40 vendu sous la dénomination commerciale "KESTER WAX K82H" par la société Koster Keunen.

Comme cires de silicone, on peut citer, par exemple, les cires de silicone polyéther, les alkyl ou alkoxy-diméthicones ayant de 16 à 45 atomes de carbone, telle que le poly(méthylalkyldiméthylsiloxane) commercialisé sous la dénomination DC 2493 par la société Dow Corning (nom CTFA : C30-45 alkyl methicone).

Selon un mode préféré de réalisation de l'invention, la composition contient comme cire, de la cire de polyéthylène, ou mieux un mélange de cire de polyéthylène et d'huile de jojoba hydrogénée.

La composition de l'invention contient une quantité de cire(s) d'au moins 3 % et allant de préférence de 3 à 10 % et mieux de 5 à 8 % en poids par rapport au poids total de la composition.

La phase huileuse de la composition selon l'invention peut renfermer, outre la cire et l'huile éventuellement présente en mélange avec l'émulsionnant siliconé, toute sorte d'huiles et de corps gras bien connus de l'homme du métier, comme par exemple les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (huile de Purcellin, mélange de myristate d'isopropyle et d'octanoate de cétéaryle ; polyisobutylène ; palmitate d'éthyl-hexyle ; alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

La phase huileuse peut contenir, en outre d'autres constituants gras tels que les alcools gras comme l'alcool stéarylique, l'alcool cétylique et l'alcool cétéarylique, et les acides gras.

La phase huileuse est présente dans la composition selon l'invention en une quantité allant de 7 à 25 % et de préférence de 10 à 20 % en poids par rapport au poids total de la composition.

Un autre avantage de la composition selon l'invention provient de ce qu'on peut y incorporer une grande quantité d'au moins un électrolyte ou d'un mélange d'électrolytes sans nuire à la stabilité de la composition.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, l'électrolyte est un mélange de sels comprenant notamment des sels de calcium, de magnésium, et de sodium, et notamment un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte(s), lorsque la composition en contient, va en général de 0,5 à 20 % et de préférence de 2 à 10 % en poids par rapport au poids total de la composition.

La composition selon l'invention contient un milieu physiologiquement acceptable et peut constituer notamment une composition cosmétique ou dermatologique. Elle trouve son application dans un grand nombre de traitements notamment cosmétiques de la peau, y compris du cuir chevelu, des cheveux, des ongles, et/ou des muqueuses, en particulier pour le soin, le nettoyage, le maquillage et/ou la protection solaire de la peau et/ou des muqueuses, ainsi que pour la préparation d'une crème destinée au traitement de la peau, plus particulièrement de la peau grasse (apport de fraîcheur).

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une crème destinée au traitement des peaux grasses.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme actifs, on peut citer notamment, outre les électrolytes indiqués ci-dessus, les hydratants et par exemple les hydrolysats de protéines, et les polyols tels que la glycérine, les glycols comme les polyéthylène glycols, et les dérivés de sucre ; les extraits naturels ; les oligomères procyannidoliques ; les vitamines ; l'urée ; les dépigmentants tels que l'acide kojique et l'acide caféique ; les bêta-hydroxyacides tels que l'acide salicylique et ses dérivés ; les alpha-hydroxyacides tels que l'acide lactique et l'acide glycolique ; les rétinoïdes tels que les caroténoïdes ; les filtres, et leurs mélanges.

Le ou les actifs peuvent être par exemple présents en une concentration allant de 0,01 à 20 %, de préférence de 0,1 à 5 % et mieux de 0,5 à 3 % du poids total de la composition.

La composition selon l'invention peut être préparée par exemple en introduisant la phase aqueuse dans la phase huileuse et en coulant le mélange à chaud, ou bien en refroidissant le mélange tout en maintenant un malaxage très lent jusqu'au retour à la température ambiante (20-25°C). La composition a un aspect solide ou bien « granité» , c'est-à-dire qu'elle a un aspect granuleux original qui diversifie la gamme de textures ayant un effet frais.

Les exemples ci-après de compositions selon l'invention sont donnés à titre d'illustration et sans caractère limitatif. Les quantités y sont données en % en poids, sauf mention contraire.

### Exemple 1 : crème solide rafraîchissante

| *Phase A :* | |
|---|---|
| - Huile de Purcellin (cetearyl octanoate / isopropyl myristate) | 2,44 % |
| - Huile de jojoba hydrogénée | 5,5 % |
| - Cire de polyéthylène | 0,8 % |
| - Exemple 3 de US-5,412,004 à 32 % en matière active | 4,82 % |
| | (soit 1,54 % de matière active) |
| | |

| *Phase B :* | |
|---|---|
| - Hexacydométhicone | 4,44 % |
| - Huile de Purecellin (cetearyl octanoate / isopropyl myristate) | 2 % |
| | |

| *Phase C :* | |
|---|---|
| - Glycérine | 2 % |
| - Chlorure de sodium | 0,5 % |
| - Eau | qsp 100 % |

Mode opératoire : on met la phase C au bain-marie à 90°C. Par ailleurs, on fait fondre la phase A vers 80-85°C sur une plaque chauffante en remuant à la spatule, puis on la met au bain-marie. On ajoute rapidement la phase B à la phase A maintenue au bain-marie, et on verse la phase C dans le mélange obtenu sous agitation. On fait couler l'émulsion obtenue dans des coupelles, puis on laisse refroidir jusqu'à température ambiante, de préférence en couvrant la coupelle.

On obtient une crème coulée solide qui perle à la prise, c'est-à-dire que l'eau perle sur le produit quand on le prend au doigt sur la coupelle.

### Exemple 2 : crème coulée

| *Phase A :* | |
|---|---|
| - Huile de Purcellin (cetearyl octanoate / isopropyl myristate) | 2,64 % |
| - Huile de jojoba hydrogénée | 5,5 % |
| - Cire de polyéthylène | 0,8 % |
| - Abil WE09 | 1,92 % |
| | |

| *Phase B :* | |
|---|---|
| - Hexacyclométhicone | 4,59 % |
| - Huile de Purcellin (cetearyl octanoate / isopropyl myristate) | 2 % |
| | |

| *Phase C :* | |
|---|---|
| - Glycérine | 2 % |
| - Chlorure de sodium | 0,5 % |
| - Eau | qsp 100 % |

Mode opératoire : identique à celui de l'exemple 1.

On obtient une crème coulée très fraîche à l'application.

## Revendications

1. Composition solide comprenant, dans un milieu physiologiquement acceptable, une phase aqueuse dispersée dans une phase huileuse, **caractérisée en ce que** la phase aqueuse représente au moins 70 % en poids par rapport au poids total de la composition, **en ce que** l'eau représente au moins 70 % en poids par rapport au poids total de la composition et **en ce que** la composition comprend au moins un émulsionnant siliconé et au moins 3 % en poids d'au moins une cire, par rapport au poids total de la composition.

2. Composition selon la revendication 1, **caractérisée en ce que** l'émulsionnant siliconé est choisi parmi les diméthicone-copolyols, les alkyl- ou alcoxy-diméthicone copolyols, les organopolysiloxanes solides élastomères réticulés comportant au moins un groupement oxyalkyléné et leurs mélanges.

3. Composition selon la revendication précédente, **caractérisée en ce que** l'alkyldiméthiconecopolyol est choisi parmi le lauryl-diméthicone-copolyol, le cétyldiméthiconecopolyol, l'oleyldiméthicone copoylol, le stéaryldiméthicone copolyol.

4. Composition selon la revendication 2, **caractérisée en ce que** l'organopolysiloxane solide élastomère réticulé comporte au moins un groupement d'oxyéthylène.

5. Composition selon la revendication 2, **caractérisée en ce que** l'organopolysiloxane solide élastomère réticulé est obtenu par réaction d'addition et de réticulation en milieu non aqueux, en présence d'un catalyseur, d'au moins :
- un premier organopolysiloxane (i) ayant deux groupements vinyliques en position α-ω de la chaîne siliconée par molécule ; et
- un second organopolysiloxane (ii) ayant au moins un atome d'hydrogène lié à un atome de silicium par molécule et au moins un groupement oxyalkyléné.

6. Composition selon la revendication précédente, **caractérisée en ce que** le premier organopolysiloxane (i) est choisi parmi les polydiméthylsiloxanes.

7. Composition selon la revendication 5 ou 6, **caractérisée en ce que** le premier organopolysiloxane (i) est un α-ω-diméthylvinyl polydiméthylsiloxane.

8. Composition selon l'une des revendications 5 à 7, **caractérisée en ce que** le second organopolysiloxane (ii) est choisi parmi les polydiméthylsiloxanes ayant un ou plusieurs atomes d'hydrogène et un ou plusieurs groupements oxyalkylénés liés à un atome de silicium via un radical alkylène ayant de 1 à 22 atomes de carbone.

9. Composition selon l'une quelconque des revendications 5 à 8, **caractérisée en ce que** l'organopolysiloxane est sous forme d'un gel obtenu selon les étapes suivantes :
- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur de platine.

10. Composition selon l'une des revendications précédentes, **caractérisée en ce que** l'émulsionnant siliconé représente de 0,1 à 10 % en poids de matière active par rapport au poids total de la composition.

11. Composition selon l'une quelconque des revendications précédentes, **caractérisée par le fait que** la cire est choisie parmi les cires d'origine animale, les cires d'origine végétale, les cires minérales, les cires synthétiques, les cires de silicone, les huiles hydrogénées concrètes à 25°C et les esters gras concrets à 25°C.

12. Composition selon l'une quelconque des revendications précédentes, **caractérisée par le fait que** la quantité de cire(s) va de 3 à 10 % en poids par rapport au poids total de la composition.

13. Composition selon l'une quelconque des revendications précédentes, **caractérisée par le fait que** la phase huileuse est présente en une quantité allant de 7 à 25 % en poids par rapport au poids total de la composition.

14. Composition selon l'une quelconque des revendications précédentes, **caractérisée par le fait que** le rapport pondéral phase huileuse/émulsionnant siliconé est égal ou supérieur à 5.

15. Composition selon l'une quelconque des revendications précédentes, **caractérisée par le fait qu'**elle contient au moins un électrolyte.

16. Composition selon la revendication précédente, **caractérisée par le fait que** l'électrolyte est présent en une quantité allant de 0,5 à 20 % du poids total de la composition.

17. Composition selon l'une quelconque des revendications précédentes, **caractérisée par le fait qu'**elle contient au moins un actif choisi parmi les hydratants, les extraits naturels, les oligomères procyannidoliques, les vitamines, l'urée, les dépigmentants, les bêta-hydroxyacides, les alpha-hydroxyacides, les rétinoïdes, les filtres, et leurs mélanges.

18. Composition selon l'une quelconque des revendications précédentes, **caractérisée en ce qu'**elle constitue une composition cosmétique.

19. Utilisation cosmétique de la composition selon l'une quelconque des revendications 1 à 18, pour le traitement, la protection, le soin, le démaquillage et/ou le nettoyage de la peau, des lèvres et/ou des cheveux, et/ou pour le maquillage de la peau et/ou des lèvres.

20. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 18.

21. Utilisation de la composition selon l'une quelconque des revendications 1 à 18 pour la fabrication d'une crème destinée au traitement des peaux grasses.

## Patentansprüche

1. Feste Zusammensetzung, die in einem physiologisch akzeptablen Medium eine in einer Ölphase dispergierte wässerige Phase enthält; **dadurch gekennzeichnet, dass** die wässerige Phase mindestens 70 Gew.-%, bezogen auf das Gesamtgewicht der Zusammensetzung, ausmacht, dadurch, dass das Wasser mindestens 70 Gew.-%, bezogen auf das Gesamtgewicht der Zusammensetzung, ausmacht, und dadurch, dass die Zusammensetzung mindestens einen siliconierten Emulgator und mindestens 3 Gew.-% mindestens eines Wachses, bezogen auf das Gesamtgewicht der Zusammensetzung, enthält.

2. Zusammensetzung nach Anspruch 1, **dadurch gekennzeichnet, dass** der siliconierte Emulgator unter den Dimethiconcopolyolen, den Alkyl- oder Alkoxydimethiconcopolyolen, den vernetzten elastomeren festen Organopolysiloxanen, die mindestens eine alkoxylierte Gruppe aufweisen, und deren Gemischen ausgewählt ist.

3. Zusammensetzung nach dem vorhergehenden Anspruch, **dadurch gekennzeichnet, dass** das Alkyldimethiconcopolyol unter Lauryldimethiconcopolyol, Cetyldimethiconcopolyol, Oleyldimethiconcopolyol und Stearyldimethiconcopolyol ausgewählt ist.

4. Zusammensetzung nach Anspruch 2, **dadurch gekennzeichnet, dass** das vernetzte elastomere feste Organopolysiloxan mindestens eine Oxyethylengruppe enthält.

5. Zusammensetzung nach Anspruch 2, **dadurch gekennzeichnet, dass** das vernetzte elastomere feste Organopolysiloxan in einem nicht wässerigen Medium in Gegenwart eines Katalysators durch Addition und Vernetzung von zumindest den folgenden Verbindungen hergestellt wird:
- einem ersten Organopolysiloxan (i), das in α-ω-Stellung der Silicankette pro Molekül zwei Vinylgruppen besitzt, und
- einem zweiten Organopolysiloxan (ii), das pro Molekül mindestens ein an ein Siliciumatom gebundenes Wasserstoffatom und mindestens eine alkoxylierte Gruppe aufweist.

6. Zusammensetzung nach dem vorhergehenden Anspruch, **dadurch gekennzeichnet, dass** das erste Organopolysiloxan (i) unter den Polydimethylsiloxanen ausgewählt ist.

7. Zusammensetzung nach Anspruch 5 oder 6, **dadurch gekennzeichnet, dass** das erste Organopolysiloxan (i) ein α,ω-Dimethylvinylpolydimethylsiloxan ist.

8. Zusammensetzung nach einem der Ansprüche 5 bis 7, **dadurch gekennzeichnet, dass** das zweite Organopolysiloxan (ii) unter den Polydimethylsiloxanen ausgewählt ist, die ein oder mehrere Wasserstoffatome und eine oder mehrere alkoxylierte Gruppen aufweisen, die über eine Alkylengruppe mit 1 bis 22 Kohlenstoffatomen an ein Siliciumatom gebunden sind.

9. Zusammensetzung nach einem der Ansprüche 5 bis 8, **dadurch gekennzeichnet, dass** das Organopolysiloxan in Form eines Gels vorliegt, das nach den folgenden Arbeitsschritten hergestellt wird:
- (a) Vermischen des ersten und des zweiten Organopolysiloxans (i) und (ii),
- (b) Zugabe einer Ölphase zu dem Gemisch aus Schritt (a) und
- (c) Polymerisieren des ersten und des zweiten Organopolysiloxans (i) und (ii) in der Ölphase in Gegenwart eines Platinkatalysators.

10. Zusammensetzung nach einem der vorhergehenden Ansprüche, **dadurch gekennzeichnet, dass** der siliconierte Emulgator 0,1 bis 10 Gew.-% Wirkstoff, bezogen auf das Gesamtgewicht der Zusammensetzung, ausmacht.

11. Zusammensetzung nach einem der vorhergehenden Ansprüche, **dadurch gekennzeichnet, dass** das Wachs unter den Wachsen tierischen Ursprungs, den Wachsen pflanzlichen Ursprungs, Mineralwachsen, synthetischen Wachsen, Siliconwachsen, bei 25 °C festen hydrierten Ölen und bei 25 °C festen Fettestern ausgewählt ist.

12. Zusammensetzung nach einem der vorhergehenden Ansprüche, **dadurch gekennzeichnet, dass** der Mengenanteil des Wachses (der Wachse) 3 bis 10 Gew.-%, bezogen auf das Gesamtgewicht der Zusammensetzung, beträgt.

13. Zusammensetzung nach einem der vorhergehenden Ansprüche, **dadurch gekennzeichnet, dass** die Ölphase in einer Menge von 7 bis 25 Gew.-%, bezogen auf das Gesamtgewicht der Zusammensetzung, vorliegt.

14. Zusammensetzung nach einem der vorhergehenden Ansprüche, **dadurch gekennzeichnet, dass** das Gewichtsverhältnis Ölphase/siliconierter Emulgator größer oder gleich 5 ist.

15. Zusammensetzung nach einem der vorhergehenden Ansprüche, **dadurch gekennzeichnet, dass** sie mindestens einen Elektrolyten enthält.

16. Zusammensetzung nach dem vorhergehenden Anspruch, **dadurch gekennzeichnet, dass** der Elektrolyt in einer Menge von 0,5 bis 20 % des Gesamtgewichts der Zusammensetzung vorliegt.

17. Zusammensetzung nach einem der vorhergehenden Ansprüche, **dadurch gekennzeichnet, dass** sie mindestens einen Wirkstoff enthält, der unter den Hydratisierungsmitteln, natürlichen Extrakten, Procyanidol-Oligomeren, Vitaminen, Harnstoff, depigmentierenden Wirkstoffen, β-Hydroxysäuren, α-Hydroxysäuren, Retinoiden, Filtern und deren Gemischen ausgewählt ist.

18. Zusammensetzung nach einem der vorhergehenden Ansprüche, **dadurch gekennzeichnet, dass** es sich um eine kosmetische Zusammensetzung handelt.

19. Kosmetische Verwendung der Zusammensetzung nach einem der Ansprüche 1 bis 18 zur Behandlung, zum Schutz, zur Pflege, zum Abschminken und/oder zur Reinigung der Haut, der Lippen und/oder der Haare und/oder zum Schminken der Haut und/oder der Lippen.

20. Verfahren zur kosmetischen Behandlung der Haut einschließlich der Kopfhaut, der Haare und/oder der Lippen, **dadurch gekennzeichnet, dass** eine Zusammensetzung nach einem der Ansprüche 1 bis 18 auf die Haut, die Haare und/oder die Lippen aufgebracht wird.

21. Verwendung der Zusammensetzung nach einem der Ansprüche 1 bis 18 zur Herstellung einer Creme, die zur Behandlung von fettiger Haut vorgesehen ist.

## Claims

1. Solid composition comprising, in a physiologically acceptable medium, an aqueous phase dispersed in an oily phase, **characterized in that** the aqueous phase represents at least 70% by weight with respect to the total weight of the composition, **in that** water represents at least 70% by weight with respect to the total weight of the composition and **in that** the composition comprises at least one silicone emulsifier and at least 3% by weight of at least one wax with respect to the total weight of the composition.

2. Composition according to Claim 1, **characterized in that** the silicone emulsifier is chosen from dimethicone copolyols, alkyl or alkoxy dimethicone copolyols, crosslinked elastomeric solid organopolysiloxanes comprising at least one oxyalkylenated group, and their mixtures.

3. Composition according to the preceding claim, **characterized in that** the alkyl dimethicone copolyol is chosen from lauryl dimethicone copolyol, cetyl dimethicone copolyol, oleyl dimethicone copolyol or stearyl dimethicone copolyol.

4. Composition according to Claim 2, **characterized in that** the crosslinked elastomeric solid organopolysiloxane comprises at least one oxyethylene group.

5. Composition according to Claim 2, **characterized in that** the crosslinked elastomeric solid organopolysiloxane is obtained by an addition and crosslinking reaction in a non-aqueous medium, in the presence of a catalyst, of at least:
- one first organopolysiloxane (i) having two vinyl groups in the α,ω-position of the silicone chain per molecule; and
- one second organopolysiloxane (ii) having at least one hydrogen atom bonded to a silicon atom per molecule and at least one oxyalkylenated group.

6. Composition according to the preceding claim, **characterized in that** the first organopolysiloxane (i) is chosen from polydimethylsiloxanes.

7. Composition according to Claim 5 or 6, **characterized in that** the first organopolysiloxane (i) is an α,ω-dimethylvinylpolydimethylsiloxane.

8. Composition according to one of Claims 5 to 7, **characterized in that** the second organopolysiloxane (ii) is chosen from polydimethylsiloxanes having one or more hydrogen atoms and one or more oxyalkylenated groups bonded to a silicon atom via an alkylene radical having from 1 to 22 carbon atoms.

9. Composition according to any one of Claims 5 to 8, **characterized in that** the organopolysiloxane is in the form of a gel obtained according to the following stages:
- (a) mixing the first and second organopolysiloxanes (i) and (ii);
- (b) adding an oily phase to the mixture of the stage (a);
- (c) polymerizing the first and second organopolysiloxanes (i) and (ii) in the oily phase in the presence of a platinum catalyst.

10. Composition according to one of the preceding claims, **characterized in that** the silicone emulsifier represents from 0.1 to 10% by weight of active material with respect to the total weight of the composition.

11. Composition according to any one of the preceding claims, **characterized in that** the wax is chosen from waxes of animal origin, waxes of vegetable origin, mineral waxes, synthetic waxes, silicone waxes, hydrogenated oils which are solid at 25°C and fatty esters which are solid at 25°C.

12. Composition according to any one of the preceding claims, **characterized in that** the amount of wax (es) ranges from 3 to 10% by weight with respect to the total weight of the composition.

13. Composition according to any one of the preceding claims, **characterized in that** the oily phase is present in an amount ranging from 7 to 25% by weight with respect to the total weight of the composition.

14. Composition according to any one of the preceding claims, **characterized in that** the oily phase/silicone emulsifier ratio by weight is equal to or greater than 5.

15. Composition according to any one of the preceding claims, **characterized in that** it comprises at least one electrolyte.

16. Composition according to the preceding claim, **characterized in that** the electrolyte is present in an amount ranging from 0.5 to 20% of the total weight of the composition.

17. Composition according to any one of the preceding claims, **characterized in that** it comprises at least one active principle chosen from moisturizing agents, natural extracts, procyanidol oligomers, vitamins, urea, depigmenting agents, β-hydroxy acids, α-hydroxy acids, retinoids, screening agents and their mixtures.

18. Composition according to any one of the preceding claims, **characterized in that** it constitutes a cosmetic composition.

19. Cosmetic use of the composition according to any one of Claims 1 to 18 for treating, protecting, caring for, removing make-up from and/or cleansing the skin, lips and/or hair and/or for making up the skin and/or lips.

20. Process for the cosmetic treatment of the skin, including the scalp, hair and/or lips, **characterized in that** a composition according to any one of Claims 1 to 18 is applied to the skin, hair and/or lips.

21. Use of the composition according to any one of Claims 1 to 18 in the manufacture of a cream intended for the treatment of greasy skin.
